# EUROPEAN PATENT SPECIFICATION

(11) **EP 4 429 972 B1**
(45) Date of publication and mention of the grant of the patent: **23.04.2025**
(21) Application number: 22813927.5
(22) Date of filing: 03.11.2022
(51) Int. Cl.: B65D 75/58

(54) **DUAL-UNIT PACK**
DOPPELEINHEITSPAKET
BLOC À DEUX UNITÉS

(30) Priority: 12.11.2021 WO PCT/CN2021/130407; 01.12.2021 EP 21211540
(43) Date of publication of application: 18.09.2024
(73) Proprietor: Unilever IP Holdings B.V., 3013 AL Rotterdam (NL); Unilever Global IP Limited, Wirral, Merseyside CH62 4ZD (GB)
(72) Inventor: MA, Siwen, 6708 WH Wageningen (NL); WANG, Boxi, 6708 WH Wageningen (NL); YANG, Fan, 6708 WH Wageningen (NL)
(74) Representative: James, Helen Sarah
(86) International application number: PCT/EP2022/080649
(87) International publication number: WO 2023/083683

(56) References cited:
- EP-A2- 0 178 918
- WO-A1-93/12009
- DE-A1- 2 751 078
- DE-U1- 20 103 336
- DE-U1- 202004 004 857
- US-A- 2 517 027

## Description

### Field of the Invention

The present invention is in the field of packaging, in particular it relates to blister packaging for at least two different consumer products.

### Background of the Invention

Blister packs are pre-formed packaging, typically plastic, used for consumer goods, foods, and for pharmaceuticals comprising a cavity (or blister) made from a formable material with a paper or aluminum backing. Blister packs are commonly used as unit dose packaging for pharmaceutical tablets, capsules or lozenges such that the consumer removes the backing to access the product retained in the cavity.

These known blister packs comprise cavities (or blisters) on one side and the backing on the other side. If the product inside the cavity is a solid product (e.g. tablets, capsules or lozenges), it may be possible to push the product through the backing. Yet, for many personal care products, home care products and some food products, especially products which have a liquid, gel or cream-like consistency, in order to access the product, the consumer needs to remove the backing and dig out the contents by hand which can be messy, unsanitary and difficult for some consumers. Another drawback of the backing on traditional blister packs is that removing the backing typically requires peeling and detaching the aluminum or paper backing. This can be quite difficult and challenging for consumers with larger hands and/or certain medical conditions including, but not limited to arthritis, Dupuytren's Disease and carpal tunnel syndrome.

A sachet is another similar packaging option for supplying unit-doses of consumer products. Unlike traditional blister packs, some sachets may allow the consumer to squeeze the sachet to remove the product. However, while sachets do not require peeling a backing (as with a traditional blister pack), they do require the consumer to tear or rip open the sachet via a tab which can be equally as challenging to open for consumers with larger hands and/or certain medical conditions including, but not limited to arthritis, Dupuytren's Disease and carpal tunnel syndrome.

In addition, in many instances, blister packs, sachets and other small packets are only used to supply a unit dose of a single product. However, for some consumer products, it is desired to be able to use a unit dose packaging format to deliver more than one product, and especially to keep the products separate such that they are only mixed immediately before use in order to preserve the freshness of each product prior to use as well as avoid or limit any potential interactions with certain ingredients in the products.

Hence, there remains a need to have an easy opening blister pack that provides convenient packaging, and at the same time provides an option to keep different products or formulae separate until they are desired to be used together.

Therefore, one aspect of the present invention is to provide packaging that can contain at least two different consumer products which are intended to be used together but are keep separate and are only mixed immediately before use, which can preserve the freshness of each product prior to use as well as avoid or limit any potential interactions between the products which could render each product, or the combination of the products, to be less potent, stable, effective or desirable to the consumer.

It is yet another object of the present invention to provide easy and convenient to open packaging which eliminates the need to remove backings or tear open tabs and only requires a single hand to easily and efficiently discharge the contents from the packaging.

DE 20103336 U1(KLOCKE VERPACKUNGS SERVICE) discloses deep-drawn pack, in particular sample pack, consisting of a base film with at least one deepdrawn cup for the filling and a sealed cover film, with a predetermined breaking point in a breakoff nose to form at least one removal opening for the filling, and with an applicator for applying the filling to a surface, characterized that the elastic applicator designed to be permeable to the filling material covers the deep-drawn pack in the area of the predetermined breaking point at least to such an extent that the filling material is dispensed exclusively through the Applicator. However, it does not specifically disclose the dual blister pack with a second cavity having at least two protrusions.

DE 202004004857 U1 (KLOCKE VERPACKUNGS SERVICE) discloses the packaging, e.g., in the form of a break-off packaging, has in its dispensing region, which may be designed as a nozzle or unsealed region for guiding of the product, a predetermined break point, which upon activation, exposes a discharge opening for the medium. Fixed above the predetermined break point is a preferably absorbent applicator, e.g., of a foamed material, nonwoven fabric, or felt. However, it does not specifically disclose the dual blister pack with a second cavity having at least two protrusions.

DE 2751078A1 (BLASY LUDWIG) discloses composite container assembly for flowable, especially pasty materials e.g., mustard, mayonnaise, or finely divided solids, comprises 2 containers such as bags, sachets or pouches, with deformable walls, which communicate by means of a flexible connecting duct. It is not the dual blister pack which has two separate cavities to avoid or limit any potential interactions between the products.

EP 0178918 A2 discloses a dispenser package for flowable substances of the type where the flowable product is contained within a flexible pouch adhered to a relatively stiff sheet material which is opened along a fault line or fault pattern with one hand by folding the stiff ends toward one another into a "V" shape, wherein the flexible product-containing pouch is formed with a shallow duct or channel area directly behind the fault line connecting two laterally spaced pockets located on either side of the fault pattern to eliminate squirting or "spitting" of the flowable product upon rupture of the fault line or fault pattern. It is not the dual blister pack which has two separate cavities to avoid or limit any potential interactions between the products.

### Summary of the Invention

In a first aspect, the present invention relates to a dual-unit pack as set out in claim 1.

In another aspect, the present invention provides for a method of using the dual-unit pack, as described herein.

In another aspect, the present invention provide use of the dual-unit pack, as described herein, for packaging at least two different consumer products.

These and other aspects, features and advantages will become apparent to those of ordinary skill in the art from a reading of the following detailed description of the preferred embodiments and the accompanying drawings. For the avoidance of doubt, any feature of one aspect of the present invention may be utilized in any other aspect of the invention.

### Brief Description of the Drawings

Fig. 1 is a perspective view of the dual-unit pack as described herein.
Fig. 2 is a side view of the dual-unit pack as described herein.
Fig. 3 is a back view of the dual-unit pack as described herein.
Fig. 4 is a top plan view of the dual-unit pack as described herein.
Fig. 5 is a top plan view of a magnified section of a part of the folding region of the dual-unit pack as described herein.
Fig. 6 is a side view of the dual-unit pack as described herein.

### Detailed Description of the Invention

The word "comprising" is intended to mean "including" but not limited to the recited steps or options. In other words, the listed steps or options need not be exhaustive.

The use of any exemplary language, e.g., "such as", is merely intended to better illuminate the invention and does not in any way limit the scope of the invention otherwise claimed.

Unless otherwise indicated, all numbers in this description indicating amounts of material or conditions of reaction, physical properties of materials and/or use are to be understood as modified by the word "about".

Numerical ranges expressed in the format "from x to y" are understood to include x and y. When for a specific feature multiple preferred ranges are described in the format "from x to y", it is understood that all ranges combining the different endpoints are also contemplated.

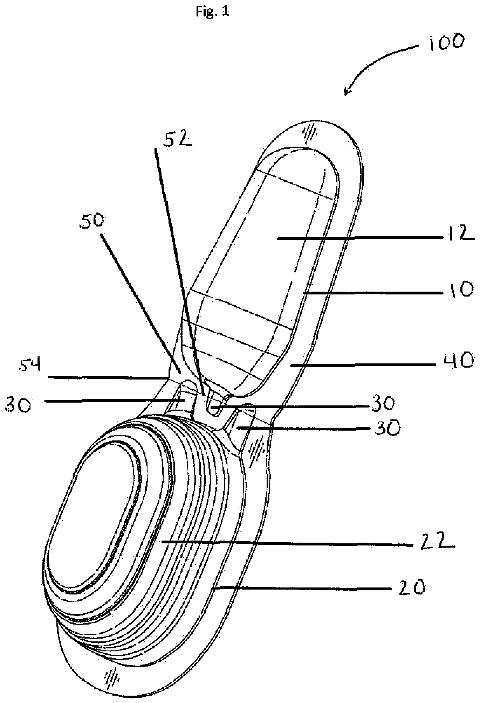

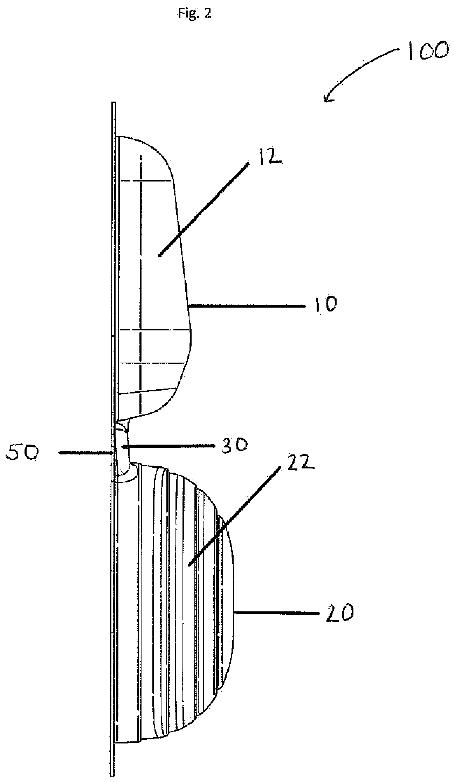

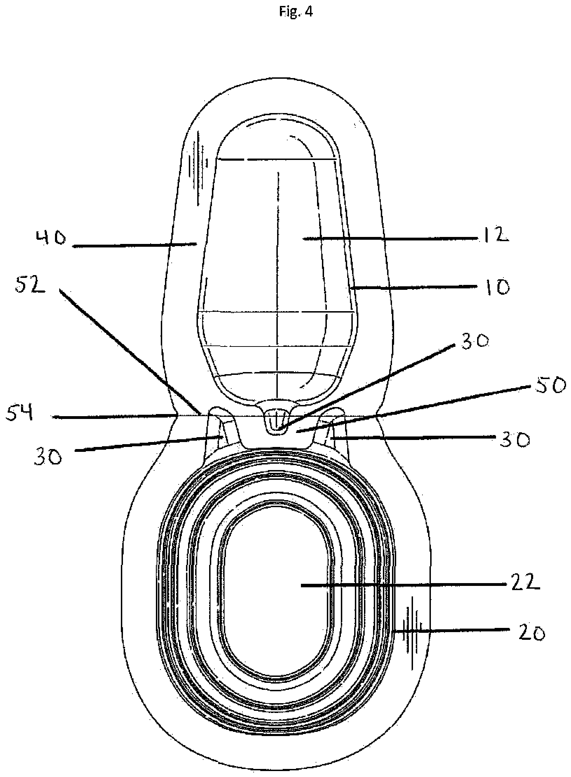

As shown in Figs. 1-6, the present invention is directed to a dual-unit pack (100) having a first blister pack (10) positioned on a formable film (40) having a first cavity (12) with at least one protrusion (30); a second blister pack (20) positioned on the same formable film (40) having a second cavity (22) with at least two protrusions (30); and a folding region (50) with a line of weakness (52) positioned in between the first and second blister packs (10, 20), wherein a backing (15) seals the first cavity (12) and the second cavity (22). The first cavity (12) and the second cavity (22) each retain a different consumer product. The line of weakness (52) intersects each of the protrusions (30).

In an embodiment, the present invention is directed to a dual-unit pack (100) having a first blister pack (10) positioned on a same formable film (40) having a first cavity (12) with at least one protrusion (30); a second blister pack (20) positioned on the formable film (40) having a second cavity (22) with at least two protrusions (30); and a folding region (50). The folding region (50) is positioned in between the first and the second blister packs (10, 20), separating the first and the second cavities (12, 22), but including the protrusions (30) and a line of weakness (52) positioned perpendicular to the longitudinal axis of the first and the second blister packs (10, 20), and parallel to the plane of the formable film (40). The line of weakness (52) in the folding region (50) intersects each of the protrusions (30). Optionally, the folding region can include die-cut portions (54) positioned at each end of the line of weakness (52). Each of the first cavity and the second cavity is sealed by a backing (15), which may be a continuous backing or separate backing. The first cavity and the second cavity each retain a different consumer product.

In an embodiment, the present invention is directed to a dual-unit pack (100) having a first blister pack (10) positioned on a same formable film (40) having a first cavity (12) with at least one protrusion (30); a second blister pack (20) positioned on the formable film (40) having a second cavity (22) with at least two protrusions (30); and a folding region (50). The second cavity (22) may optionally have divider wall creating two separate compartments each with a protrusion (30). The folding region (50) is positioned in between the first and the second blister packs (10, 20), separating the first and the second cavities (12, 22), but including the protrusions (30) and a line of weakness (52) positioned perpendicular to the longitudinal axis of the first and the second blister packs (10, 20), and parallel to the plane of the formable film (40). The line of weakness (52) in the folding region (50) intersects each of the protrusions (30). Optionally, the folding region can include die-cut portions (54) positioned at each end of the line of weakness (52). Each of the first cavity and the second cavity is sealed by a backing (15), which may be a continuous backing or separate backing. The first cavity (12) and the second cavity (22) each retain a different consumer product. Optionally, if the second cavity has two separate compartments, each of the first cavity and the compartments retain a different consumer product, and thus the dual-unit pack (100) may contain three different consumer products.

In an embodiment, the present invention is directed to a dual-unit pack (100) having a first blister pack (10) positioned on a formable film (40) having a first cavity (12) with at least one protrusion (30); a second blister pack (20) positioned on the same formable film (40) have two separate and distinct cavities each with a protrusion (30); and a folding region (50). The folding region (50) is positioned in between the first and the second blister packs (10, 20), separating the cavities but including the protrusions (30) and a line of weakness (52) positioned perpendicular to the longitudinal axis of the first and the second blister packs (10, 20), and parallel to the plane of the formable film (40). The line of weakness (52) in the folding region (50) intersects each of the protrusions (30). Optionally, the folding region can include die-cut portions (54) positioned at each end of the line of weakness (52). Each of the cavities is sealed by a backing (15), which may be a continuous backing or separate backing. Each cavity retains a different consumer product. Each cavity may retain a different consumer product with different viscosity or fluidity. It is preferred that the dual-unit pack of the present invention does not comprise an applicator, which is preferably a sponge.

As described herein, a blister pack refers to a pre-formed packaging used for consumer products having a cavity (otherwise known as a pocket or blister) made from a formable film (40) sealed with a backing (15). As described herein, the first and second blister packs (10, 20) are separate from one another and each can contain a consumer product, preferably a different consumer product.

A consumer product, as described herein, refers to a liquid or semi-solid product, for example, a liquid, a gel, an emulsion, or a cream. In an embodiment, the consumer product is a home care product, a personal care product or a food product. A home care product may be, for example, laundry detergents, fabric softeners, dishwashing detergents and other household cleaning compositions. A personal care product may be, for example, hair products (such as shampoo, conditioner, hair masks, hair oils, hair styling products), face masks, soaps (such as hand wash, face wash, and/or body wash/shower gel), personal care compositions (such as cosmetic compositions, face lotions, creams or emulsions, or body lotions, creams or emulsions), mouthwash, intimate wash and shaving gel. A food product may be, for example, food concentrates, sauces, and dressings.

In an embodiment, each blister pack of dual-unit pack (100), as described herein, has a different consumer product, and the consumer products in each blister pack are intended to be used together, e.g., both blister packs can contain a personal care product, yet each blister has a different personal care product. Such different personal care products may have different viscosity or fluidity. By keeping the consumer products in separate blister packs (or cavity and/or compartments thereof), it is possible to preserve the freshness, prevent interaction of some of the consumer products, and maintain the stability of each of the consumer products. As used herein, "stability" or "stable" refers to consumer products that do not discolor or decompose, do not have any foul odors, and retain potency.

In an embodiment, the first blister pack (10) may contain a shampoo and the second blister pack (20) may contain a conditioner, or the first blister pack (10) may contain a conditioner and the second blister pack (20) may contain a hair mask, or the first blister pack (10) may contain a hair mask and the second blister pack (20) may contain a hair oil.

In an embodiment, each of the blister packs (or cavity and/or compartment thereof) include a different personal care composition. The active and/or functional ingredient(s) that may be included in the personal care compositions, include but are not limited to: skin benefit agents for even skin tone and reducing age spots, antioxidants, vitamins, minerals, emollients, humectants, sunscreens, anti-irritants, and/or exfoliating agents. In an embodiment, each personal care composition included in the dual-unit pack (100) only has a single active and/or functional ingredient. In an embodiment, one or more of the personal care compositions included in the dual-unit pack (100) may comprise one or more active and/or functional ingredient(s) so long as the composition is stable, as defined herein.

For example, at least one blister pack (or cavity and/or compartment thereof) can include a composition comprising a resorcinol. A "resorcinol", as used herein, is a dihydroxy phenol compound (i.e., 1,3-dihydroxybenzene) characterized by alcohol groups (-OH) at positions 1 and 3. The chemical structure of resorcinols may be modified, resulting in substituted resorcinols characterized by at least one substituent in the 2, 4, 5 or 6 position. It is preferred that the resorcinol comprises at least one substituent comprising 5 to 11 carbon atoms, preferably, 5 to 9 carbon atoms. It is particularly preferred that at least one substituent comprises an alkyl group, more preferably, an unsaturated alkyl group. Examples of resorcinols include: 4-ethylresorcinol, 4-hexylresorcinol, 4-phenylethylresorcinol, 4-cyclopentyl resorcinol, 4-cyclohexylresorcinol, 4-octylresorcinol, and mixtures thereof. In an embodiment, when it is desired to use more than one resorcinol, these compounds may be included in the same or different personal care composition.

In an embodiment, at least one blister pack (or cavity and/or compartment thereof) can include a composition comprising a retinoic acid precursor or derivative, including but not limited to: retinyl esters, retinol, retinal, and mixtures thereof. Examples of retinyl ester include: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecandate, retinyl taurate, retinyl tridecanoate, retinyl myristate, retinyl pentadecanoate, retinyl heptadeconoate, retinyl stearate, retinyl isostearate, retinyl nonadecanoate, retinyl arachidonate, retinyl behenate, retinyl linoleate, retinyl oleate, and mixtures thereof. In an embodiment, when it is desired to use more than one retinoic acid precursor or derivative, these compounds may be included in the same or different composition.

In an embodiment, at least one blister pack (or cavity and/or compartment thereof) can include a composition comprising vitamin C and/or a derivative thereof.

In an embodiment, at least one blister pack (or cavity and/or compartment thereof) can include a composition comprising vitamin D and/or a derivative thereof.

In an embodiment, at least one blister pack (or cavity and/or compartment thereof) can include a composition comprising vitamin E and/or a derivative thereof.

In an embodiment, at least one blister pack (or cavity and/or compartment thereof) can include a composition comprising at least one functionalized heteroaromatic compound including but not limited to: nicotinic acid, niacinamide, nicotinate, picolinamide, picolinic acid, or derivatives and mixtures thereof.

In an embodiment, at least one blister pack (or cavity and/or compartment thereof) can include a composition comprising a conjugated linoleic acid ("CLA") and/or a derivative thereof.

In an embodiment, the resorcinol and the retinoic acid precursor or derivative are not included in the same personal care composition, e.g., the at least one resorcinol is not included in the composition in the same blister pack (or cavity and/or compartment thereof) as the retinoic acid precursor or derivative. Instead, the resorcinol and the retinoic acid precursor or derivative are freshly mix right before use.

As described herein, the formable film (40) refers to a material, such as a plastic, in which the cavities are formed. In an embodiment, the cavities are formed from the same piece of formable film (40). For example, the cavities are made of the formable film (40) running continuously from the first cavity (12) to the second cavity (22). The overall shape of the formable film can be any suitable shape.

In an embodiment, the formable film (40) is a thermoplastic polymeric material, such as polyvinylchloride (homopolymer or copolymer), polyester, polypropylene, polyethylene, fluorocarbon polymers, copolymers and terpolymers, laminates, resins and coatings of such materials, and such materials having modifying components like plasticizers therein. In an embodiment, the plastic is polyethylene terephthalate/polyethylene (PET/PE) laminate, polyacrylonitrile and polystyrene resin, ethylene-vinyl alcohol co-polymers and combinations thereof. In an embodiment, the plastic is biodegradable or recyclable plastic including machine-direction orientation (MDO) films, in particular MDO films made from polyethylene, biaxially oriented polypropylene (BOPP)/cast unoriented polypropylene (CPP) films. In an embodiment, the plastic is biodegradable and recyclable. In an embodiment, the formable film (40) is a metal foil, optionally with a heat-sealing layer, e.g., a hot-melt adhesive or glue coating. In an embodiment, the formable film (40) is a paper. In an embodiment, a thickness of the formable film (40) is about 0.1 mm to about 2 mm, preferably from about 0.1 mm to about 1.5 mm, more preferably from about 0.1 mm to about 1 mm, and even more preferably from about 0.1 mm to about 0.8 mm.

In an embodiment, the first cavity (12) and second cavity (22) can be any suitable shape for retaining a consumer product. In an embodiment, the first cavity (12) and second cavity (22) can be the same or a different shape. In an embodiment, the shape of the first cavity (12) and the second cavity (22) is suitable to be placed into a consumer's hand so that the consumer can simultaneously squeeze each cavity (12, 22) with a single hand once the line of weakness is snapped.

In an embodiment, the first cavity (12) and second cavity (22) have the same fillable volume. In an embodiment, the first cavity (12) and second cavity (22) have a different fillable volume.

In an embodiment, at least one of the blister packs may contain more than one separate and distinct cavity (not shown), where each cavity is connected to at least a protrusion (30) such that when the line of weakness (52) is snapped, the consumer can discharge the contents from each blister pack (and the cavities therein) by simultaneously squeezing the cavities of the first and the second blister packs.

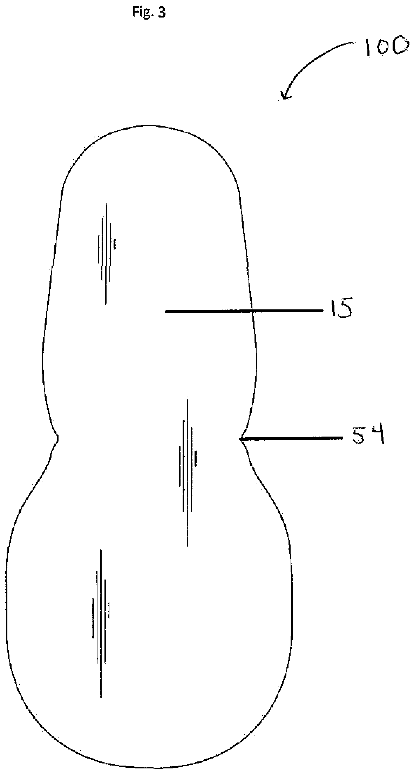

As described herein, and especially as shown in Fig. 3, the backing (15) is a material which seals the cavities (12, 22) of the blister packs (10, 20) such that the contents cannot exit the cavity. Any known materials commercially available can be used for the backing (15) such as aluminum, plastic, and combination thereof. In an embodiment, the plastic is a thermoplastic polymeric material, including polyvinylchloride (homopolymer or copolymer), polyester, polypropylene, polyethylene, fluorocarbon polymers, copolymers and terpolymers, laminates and coatings of such materials, and such materials having modifying components like plasticizers therein. In an embodiment the backing is polyethylene terephthalate/aluminum (PET/AL) laminate. In an embodiment, the backing is made of a biodegradable and/or recyclable material.

In an embodiment, the backing (15) is a different material than that of the formable film (40). In an embodiment, the backing (15) and the formable film (40) are the same material.

In an embodiment, a thickness of the backing (15) is about 0.1 mm to about 2 mm, preferably from about 0.1 mm to about 1.5 mm, more preferably from about 0.1 mm to about 1 mm, and even more preferably from about 0.1 mm to about 0.8 mm.

In an embodiment, the backing (15) may have a perforated, scored or partially cut line (not shown) that mirrors the line of weakness (52). In an embodiment, if the backing (15) has a perforated, scored or partially cut line, the depth of this cut line is about 1% to 60% of the thickness of the backing (15), preferably about 5% to 50%, and more preferably about 5% to about 20%.

In an embodiment, the backing (15) is a continuous backing such that the backing (15) continuously runs over the cavities (12, 22) to cover and seal each of the cavities (12, 22). In an embodiment, a continuous backing may also continuously run over the length and width of the formable film (40) (e.g., cover the surface area of the formable film) as shown in Fig. 3.

In an embodiment, the backing (15) is a separate backing such that material is used to distinctly cover each cavity separately, e.g., the backing for the first cavity (12) is separate from the backing of the second cavity (22), where each backing may be made of the same or a different material.

Unlike traditional blister packs, the backing (15) (either the continuous backing or the separate backing) is not intended to be removed by the consumer (e.g., by peeling and detaching the backing).

The protrusions (30) of the first and the second blister packs (10, 20) are the narrow extension channels positioned at the proximal end of each cavity (12, 22), which may be any suitable dimension for discharging a consumer product retained inside the cavity once the line of weakness (52) is snapped. The use of the protrusions (30) as described herein, eliminates tabs or other opening mechanisms that require the consumer to rip or tear, e.g., a tab, or peel a backing to release or access the contents of the blister pack.

In an embodiment, each of the first and the second blister packs (10, 20) has least two protrusion (30). In an embodiment, the first and the second blister packs (10, 20) have the same or different number of protrusions (30). In an embodiment, the first blister pack (10) has one protrusion (30), and the second blister pack (20) has two protrusions (30). In an embodiment, the first blister pack (10) has two protrusions (30), and the second blister pack (20) has two protrusions (30). The line of weakness (52), as described herein, intersects each of the protrusions (30). Each of the protrusions (30) is connected to a cavity in one of blister packs.

In an embodiment, if a blister pack has more than one protrusion (30), the cavity may have a divider wall that runs from the proximal end of the cavity to the distal end of the cavity (not shown), such that the divider wall creates two separate and distinct compartments within the cavity and each compartment has a protrusion. In this manner, it is possible for the dual-unit pack (100) to hold at least three different consumer products which are all kept separate until the consumer intends to use the products. In an embodiment, each of the first and the second blister pack has more than one protrusion (30), and each of the first and the second cavity has a divider wall creating two compartments in the first cavity and two compartments in the second cavity such that the dual-unit pack (100) can hold up to four different consumer products which are all kept separate until the consumer intends to use the products.

In an embodiment, the divider wall(s) may be made of the same or different material as the formable film (40).

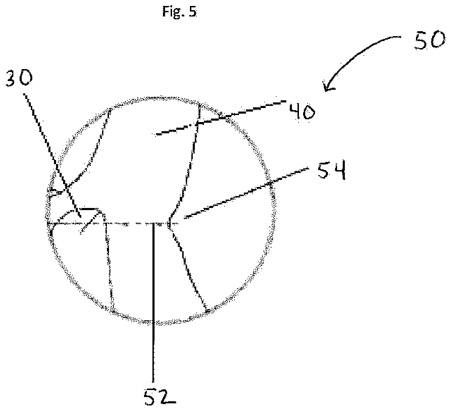

The dual-unit pack (100) has a folding region (50). The folding region (50) is the area of the formable film (40) that is in between the first and the second blister pack (10, 20) and separates the first cavity (12) from the second cavity (22) but includes the protrusions (30). The folding region, as partially shown in Fig. 5, includes a line of weakness (52) which intersects the protrusions (30) of the first and the second blister packs (10, 20).

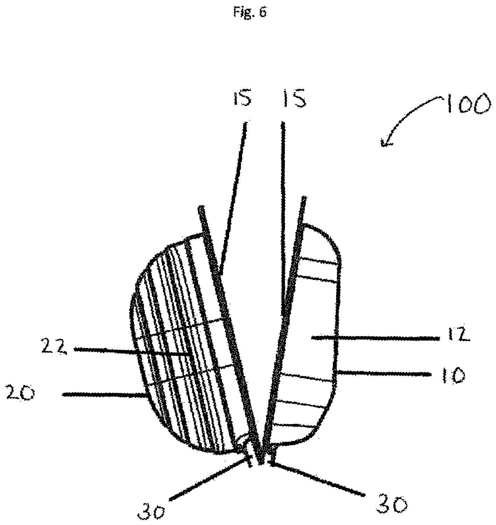

The line of weakness (52) refers to a scored, perforated and/or partially cut line in the formable film (40) which is positioned in the folding region (50) (e.g., between the first blister pack (10) and the second blister pack (20) of the dual-unit pack (100)), and intersects each protrusion (30), which permits folding, but not separation of the first and second blister packs (10, 20). In an embodiment, the line of weakness (52) is perpendicular to the longitudinal axis of the first and the second blister packs (10, 20) and parallel to the plane of the formable film (40) prior to snapping the line of weakness (52). In an embodiment, a depth of the line of weakness is about 10% to 90% of the thickness of the formable film (40), preferably about 15% to 85%, and more preferably about 20% to about 80%. When the line of weakness (52) is snapped, as shown in Fig. 6, this refers to the consumer folding the dual-unit pack (100) at the scored, perforated and/or partially cut line.

Prior to snapping the line of weakness (52) in the folding region (50), the protrusions (30) are sealed, such that the consumer products cannot be discharged from the protrusions (30). In addition, prior to snapping the line of weakness (52), each blister pack (10, 20) is positioned along the same plane, as shown in Figs. 1-4. However, once the consumer snaps the line of weakness (52) by folding the dual-unit pack (100) along the line of weakness (52), as shown in Fig. 6, this opens each of the protrusions (30) so that it is possible to squeeze the first and the second blister packs (10, 20) simultaneously to discharge the consumer product in each blister pack. In an embodiment, the folding along the line of weakness (52) is possible without the use of a tool, and most preferably by hand, such that the backing (15) on each side of the line of weakness is brought together, e.g., backing portion sealing the first cavity (12) (on one side of the line of weakness (52)) faces or confronts the backing portion sealing the second cavity (22) (on the other side of the line of weakness (52)), and optionally meets and/or touches such that each of the first and second cavities (12, 22) face outward. In this manner, it is possible for the consumer to hold the dual-unit pack (100) in a single hand and squeeze the first and second cavities (12, 22) of the first and the second blister packs (10, 20) simultaneously allowing for the consumer products to be discharged from each of the protrusions (30). In an embodiment, when each of the cavities (12, 22) retain food products, it may be possible to insert a portion of the dual-unit blister pack (100) with the protrusions (30) and squeeze the contents into the consumer's mouth to consume food product quickly and easily.

In an embodiment, the folding region (50) also has at least two die-cut portions (54), preferably positioned by each end (or at each endpoint) of the line of weakness (52). These die-cut portions (54) may be helpful to guide the consumer to fold the dual-unit pack (100) as well as control the manner of folding such that the backing (15) on each side of the line of weakness is brought together. In an embodiment, each die-cut portion (54) is a concave die-cut, preferably having an R (or radius of curvature of a concave portion which is the distance from the vertex to the center of curvature) from about 0.2 mm to about 6 mm, preferably about 0.4 mm to about 5 mm, and more preferably from about 0.5 mm to about 4 mm.

In another aspect, the present invention relates to a method for using the dual-unit pack (100) as described herein. In an embodiment, the method comprises snapping the line of weakness (52) in the folding region (50); folding the first and second blister packs (10, 20) together such that the backing on each side of the line of weakness is brought together with the first and the second cavities (12, 22) facing outward; and squeezing the first and the second cavities (12, 22) of the first and the second blister packs (10, 20) simultaneously to release the consumer products from the protrusions. It is possible to squeeze the products on, for example, a surface, into/on the consumer's hand, and for food products, into the consumer's mouth. The consumer can continue to squeeze the cavities until each cavity is empty.

In the context of the present invention, the reference to empty/emptied blister packs typically means the consumer products in the first and/or the second blister packs (10, 20) has been used, particularly squeezed from the blister pack from the protrusions (30).

In yet another aspect, the present invention relates to use of the dual-unit pack, as described herein, for packaging at least two different consumer products, or at least three different consumer products, or at least four different consumer products.

In yet another aspect, the present invention relates to use of the dual-unit pack, as described herein, for packaging at least two different personal care products. In yet another aspect, the present invention relates to use of the dual-unit pack, as described herein, for packaging at least two different home care products. In yet another aspect, the present invention relates to use of the dual-unit pack, as described herein, for packaging at least two different food products.

It should be understood, of course, that the specific forms of the invention herein illustrated and described are intended to be representative only as certain changes may be made therein without departing from the clear teachings of the disclosure. Accordingly, reference should be made to the following appended claims in determining the full scope of the invention.

### Examples

This example demonstrated the impact of numbers of protrusions on the feasibility of squeezing the different products out of the dual-blister pack and mixed uniformly. Three different dual-blister backs (A-2) with consumer products are used as showed below:

### Dual-blister pack A:

Comprising a first blister pack (10) positioned on a formable film (40) having a first cavity (12) with one protrusion; a second blister pack (20) positioned on the same formable film (40) having a second cavity (22) with one protrusion (30); and a folding region (50) positioned in between the first blister pack (10) and second blister pack (20), wherein the folding region (50) has a line of weakness (52) which intersects the protrusions (30) of the first blister pack (10) and the second blister pack (20); and wherein a backing (15) seals the first cavity (12) and the second cavity (22).

The first cavity (12) is filled with hair oil and the second cavity (22) is filled with hair mask.

### Dual-blister pack 1:

Comprising a first blister pack (10) positioned on a formable film (40) having a first cavity (12) with one protrusion; a second blister pack (20) positioned on the same formable film (40) having a second cavity (22) with two protrusions (30); and a folding region (50) positioned in between the first blister pack (10) and second blister pack (20), wherein the folding region (50) has a line of weakness (52) which intersects the protrusions (30) of the first blister pack (10) and the second blister pack (20); and wherein a backing (15) seals the first cavity (12) and the second cavity (22).

The first cavity (12) is filled with hair oil and the second cavity (22) is filled with hair mask.

### Dual-blister pack 2:

Comprising a first blister pack (10) positioned on a formable film (40) having a first cavity (12) with two protrusions; a second blister pack (20) positioned on the same formable film (40) having a second cavity (22) with two protrusions (30); and a folding region (50) positioned in between the first blister pack (10) and second blister pack (20), wherein the folding region (50) has a line of weakness (52) which intersects the protrusions (30) of the first blister pack (10) and the second blister pack (20); and wherein a backing (15) seals the first cavity (12) and the second cavity (22).

The first cavity (12) is filled with hair oil and the second cavity (22) is filled with hair mask.

### Test method:

Try to use a single hand to efficiently discharge the contents from the dual-blister packs and mix the contents from different cavity uniformly and observe the feasibility.

The observed result is summarized below:
For Dual-blister pack A: Product in first cavity (12) can be squeezed out easily and smoothly; there is still some product left in second cavity (22).

For Dual-blister pack 1: Products in first cavity (12) and second cavity (22) can be squeezed easily through protrusions and mixed uniformly.

For Dual-blister pack 2: Products in first cavity (12) and second cavity (22) can be squeezed easily through protrusions and mixed uniformly.

The result above demonstrates that dual blister pack of the present invention (Samples 1-2) provide desired feasibility of squeezing samples smoothly by one single hand and the products contained can be mixed uniformly, while the dual blister pack outside the present invention (Sample A) cannot.

## Claims

1. A dual-unit pack (100) comprising:
a first blister pack (10) positioned on a formable film (40) having a first cavity (12) with at least one protrusion (30);
a second blister pack (20) positioned on the same formable film (40) having a second cavity (22) with at least one protrusion (30); and
a folding region (50) positioned in between the first blister pack (10) and second blister pack (20),
wherein the folding region (50) has a line of weakness (52) which intersects the protrusions (30) of the first blister pack (10) and the second blister pack (20); and
wherein a backing (15) seals the first cavity (12) and the second cavity (22); wherein the second cavity (22) has at least two protrusions (30); wherein the protrusion is the narrow extension channels positioned at the proximal end of each cavity (12, 22), which may be any suitable dimension for discharging a consumer product retained inside the cavity once the line of weakness (52) is snapped.

2. The dual-unit pack (100) of claim 1, wherein the first blister pack (10) has two protrusion (30), and the second blister pack (20) has two protrusions (30).

3. The dual-unit pack (100) of claims 1 or 2, wherein a thickness of the formable film (40) is about 0.1 mm to about 2 mm, preferably from about 0.1 mm to about 1.5 mm, more preferably from about 0.1 mm to about 1 mm, and even more preferably from about 0.1 mm to about 0.8 mm.

4. The dual-unit pack (100) of claim 3, wherein a depth of the line of weakness (52) is about 10% to 90% of the thickness of the formable film, preferably about 15% to 85%, and more preferably about 20% to about 80%.

5. The dual-unit pack (100) of claims 1-4, wherein the line of weakness is perpendicular to a longitudinal axis of the first and the second blister packs (10, 20) and parallel to the plane of the formable film (40).

6. The dual-unit pack (100) of any of the preceding claims further comprising two die-cuts portions (54) in the folding region (50), preferably positioned at each endpoint of the line of weakness (52).

7. The dual-unit pack (100) of claim 6, wherein the two die-cuts portions (54) are concave, preferably having an R of from about 0.2 mm to about 6 mm, preferably about 0.4 mm to about 5 mm, and more preferably from about 0.5 mm to about 4 mm.

8. The dual-unit pack (100) of any of the preceding claims, wherein the formable film (40) is a plastic.

9. The dual-unit pack (100) of any of the preceding claims, wherein the first and second cavity (12, 22) retain a consumer product, and wherein the consumer product in the first and second cavity (12, 22) are different.

10. The dual-unit pack (100) of claim 9, wherein the first cavity (12) comprises a personal care composition with at least one resorcinol and the second cavity (22) comprises a personal care composition comprising at least one retinoic acid precursor or derivative,
wherein the at least one resorcinol is selected from 4-ethylresorcinol, 4-hexylresorcinol, 4-phenylethylresorcinol, 4-cyclopentyl resorcinol, 4-cyclohexylresorcinol, 4-octylresorcinol, and mixtures thereof,
wherein the least one retinoic acid precursor or derivative is selected from retinol, retinal, retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecandate, retinyl taurate, retinyl tridecanoate, retinyl myristate, retinyl pentadecanoate, retinyl heptadeconoate, retinyl stearate, retinyl isostearate, retinyl nonadecanoate, retinyl arachidonate, retinyl behenate, retinyl linoleate, retinyl oleate and mixtures thereof, and
with the proviso that the at least one resorcinol is not included in the composition in the second cavity and the at least one retinoic acid precursor or derivative is not included in the composition in the first cavity.

11. The dual-unit pack (100) of claim 10, the first cavity (12) and/or the second cavity (22) comprises a functionalized heteroaromatic compound, preferably nicotinic acid, niacinamide, nicotinate, picolinic acid, picolinamide, and mixtures thereof.

12. The dual-unit pack (100) of claim 9, wherein the consumer product is a personal care product.

13. Use of the dual-unit pack (100) of claims 1-8 for packaging two different consumer products, preferably personal care products, even more preferably hair products or personal care compositions.

14. A method of using the dual-unit pack (100) of claims 1-8 comprising:
snapping the line of weakness (52) in the folding region (50);
folding the first and the second blister packs (10, 20) together such that the backing (15) on each side of the line of weakness is brought together, with the first and the second cavities (12, 22) facing outward; and
squeezing the first and the second cavities (12, 22) of the first and the second blister packs (10, 20) simultaneously to release a consumer product from the protrusions (30).

## Patentansprüche

1. Zweikammerpackung (100), die Folgendes umfasst:
eine erste Blisterpackung (10), die auf einer verformbaren Folie (40) positioniert ist und die einen ersten Hohlraum (12) mit wenigstens einem Vorsprung (30) aufweist;
eine zweite Blisterpackung (20), die auf derselben verformbaren Folie (40) positioniert ist und die einen zweiten Hohlraum (22) mit wenigstens einem Vorsprung (30) aufweist; und
einen Knickbereich (50), der zwischen der ersten Blisterpackung (10) und der zweiten Blisterpackung (20) angeordnet ist,
wobei der Knickbereich (50) eine Schwächungslinie (52) aufweist, die die Vorsprünge (30) der ersten Blisterpackung (10) und der zweiten Blisterpackung (20) schneidet; und
wobei eine Rückseite (15) den ersten Hohlraum (12) und den zweiten Hohlraum (22) abdichtet; wobei der zweite Hohlraum (22) wenigstens zwei Vorsprünge (30) aufweist; wobei der Vorsprung der schmale Verlängerungskanal ist, der am proximalen Ende jedes Hohlraums (12, 22) positioniert ist, der eine beliebige geeignete Abmessung zum Abgeben eines Verbraucherprodukts, das im Hohlraum enthalten ist, bis die Schwächungslinie (52) zerrissen wird, aufweisen kann.

2. Zweikammerpackung (100) nach Anspruch 1, wobei die erste Blisterpackung (10) zwei Vorsprünge (30) aufweist und die zweite Blisterpackung (20) zwei Vorsprünge (30) aufweist.

3. Zweikammerpackung (100) nach Anspruch 1 oder 2, wobei die Dicke der verformbaren Folie (40) etwa 0,1 mm bis etwa 2 mm, vorzugsweise etwa 0,1 mm bis etwa 1,5 mm, stärker bevorzugt etwa 0,1 mm bis etwa 1 mm und noch stärker bevorzugt etwa 0,1 mm bis etwa 0,8 mm beträgt.

4. Zweikammerpackung (100) nach Anspruch 3, wobei eine Tiefe der Schwächungslinie (52) etwa 10 % bis 90 %, vorzugsweise etwa 15 % bis 85 % und stärker bevorzugt etwa 20 % bis etwa 80 % der Dicke der verformbaren Folie beträgt.

5. Zweikammerpackung (100) nach Anspruch 1-4, wobei die Schwächungslinie senkrecht zur Längsachse der ersten und der zweiten Blisterpackung (10, 20) und parallel zur Ebene der verformbaren Folie (40) verläuft.

6. Zweikammerpackung (100) nach einem der vorhergehenden Ansprüche, die ferner zwei gestanzte Abschnitte (54) im Knickbereich (50) umfasst, die vorzugsweise am jeweiligen Endpunkt der Schwächungslinie (52) positioniert sind.

7. Zweikammerpackung (100) nach Anspruch 6, wobei die zwei gestanzten Abschnitte (54) konkav sind, wobei sie vorzugsweise ein R von etwa 0,2 mm bis etwa 6 mm, bevorzugt von etwa 0,4 mm bis etwa 5 mm, und stärker bevorzugt von etwa 0,5 mm bis etwa 4 mm aufweisen.

8. Zweikammerpackung (100) nach einem der vorhergehenden Ansprüche, wobei die verformbare Folie (40) ein Kunststoff ist.

9. Zweikammerpackung (100) nach einem der vorhergehenden Ansprüche, wobei der erste und der zweite Hohlraum (12, 22) ein Verbraucherprodukt enthalten und wobei das Verbraucherprodukt im ersten und im zweiten Hohlraum (12, 22) jeweils unterschiedlich ist.

10. Zweikammerpackung (100) nach Anspruch 9, wobei der erste Hohlraum (12) eine Mischung zur Körperpflege mit wenigstens einem Resorzin umfasst und der zweite Hohlraum (22) eine Mischung für die Körperpflege umfasst, die einen Retinolsäure-Precursor und/oder ein Derivat umfasst,
wobei das wenigstens eine Resorzin aus 4-Ethylresorzin, 4-Hexylresorzin, 4-Phenylethylresorzin, 4-Cylopentylresorzin, 4-Cyclohexylresorzin, 4-Octylresorzin und Mischungen davon ausgewählt wird,
wobei der Retinolsäure-Precursor und/oder das Derivat ausgewählt werden aus: Retinol, Retinal, Retinylpalmitat, Retinylformiat, Retinylacetat, Retinylpropionat, Retinylbutyrat, Retinylvalerat, Retinylisovalerat, Retinylhexanoat, Retinylheptanoat, Retinyloctanoat, Retinylnonanoat, Retinyldecanoat, Retinylundecandat, Retinyltaurat, Retinyltridecanoat, Retinylmyristat, Retinylpentadecanoat, Retinylheptadeconoat, Retinylstearat, Retinylisostearat, Retinylnonadecanoat, Retinylarachidonat, Retinylbehenat, Retinyllinoleat, Retinyloleat und Mischungen davon, und
wobei vorausgesetzt wird, dass das wenigstens eine Resorzin in der Mischung im zweiten Hohlraum nicht enthalten ist und der wenigstens eine Retinolsäure-Precursor oder das Derivat in der Mischung im ersten Hohlraum nicht enthalten ist.

11. Zweikammerpackung (100) nach Anspruch 10, wobei der erste Hohlraum (12) und/oder der zweite Hohlraum (22) eine funktionalisierte heteroaromatische Mischung, vorzugsweise Nikotinsäure, Niacinamid, Nikotinat, Picolinsäure, Picolinamid und Mischungen davon umfassen.

12. Zweikammerpackung (100) nach Anspruch 9, wobei das Verbraucherprodukt ein Körperpflegeprodukt ist.

13. Verwendung der Zweikammerpackung (100) nach Anspruch 1-8 zum Verpacken von zwei unterschiedlichen Verbraucherprodukten, vorzugsweise von Körperpflegeprodukten, stärker bevorzugt von Haarprodukten oder Körperpflege-Mischungen.

14. Verfahren zur Verwendung der Zweikammerpackung (100) nach den Ansprüchen 1-8, das die folgenden Schritte umfasst:
Einreißen der Schwächungslinie (52) im Knickbereich (50);
Zusammenklappen der ersten und der zweiten Blisterpackung (10, 20), so dass die Rückseiten (15) auf beiden Seiten der Schwächungslinie zusammengebracht werden, wobei der erste und der zweite Hohlraum (12, 22) nach außen zeigen; und
gleichzeitiges Quetschen des ersten und des zweiten Hohlraums (12, 22) der ersten und der zweiten Blisterpackung (10, 20), um aus den Vorsprüngen (30) ein Verbraucherprodukt abzugeben.

## Revendications

1. Emballage à deux unités (100) comprenant :
un premier emballage coque (10) positionné sur un film formable (40) ayant une première cavité (12) avec au moins une protubérance (30);
un deuxième emballage coque (20) positionné sur le même film formable (40) ayant une deuxième cavité (22) avec au moins une protubérance (30) ; et
une région de pliage (50) positionnée entre le premier emballage coque (10) et le deuxième emballage coque (20),
dans lequel la région de pliage (50) a une ligne de fragilité (52) qui croise les protubérances (30) du premier emballage coque (10) et du deuxième emballage coque (20) ; et
dans lequel un envers (15) scelle la première cavité (12) et la deuxième cavité (22) ; dans lequel la deuxième cavité (22) a au moins deux protubérances (30) ; dans lequel la protubérance consiste en les canaux d'extension étroits positionnés à l'extrémité proximale de chaque cavité (12, 22), pouvant avoir n'importe quelle dimension convenable pour décharger un produit de consommation retenu à l'intérieur de la cavité une fois que la ligne de fragilité (52) est rompue.

2. Emballage à deux unités (100) selon la revendication 1, dans lequel le premier emballage coque (10) a deux protubérances (30), et le deuxième emballage coque (20) a deux protubérances (30).

3. Emballage à deux unités (100) selon la revendication 1 ou 2, dans lequel l'épaisseur du film formable (40) est d'environ 0,1 mm à environ 2 mm, de préférence d'environ 0,1 mm à environ 1,5 mm, mieux encore d'environ 0,1 mm à environ 1 mm, et plus particulièrement d'environ 0,1 mm à environ 0,8 mm.

4. Emballage à deux unités (100) selon la revendication 3, dans lequel la profondeur de la ligne de fragilité (52) est d'environ 10 % à 90 %, de préférence d'environ 15 % à 85 %, et mieux encore d'environ 20 % à environ 80 % de l'épaisseur du film formable.

5. Emballage à deux unités (100) selon les revendications 1 à 4, dans lequel la ligne de fragilité est perpendiculaire à l'axe longitudinal des premier et deuxième emballages coques (10, 20) et parallèle au plan du film formable (40).

6. Emballage à deux unités (100) selon l'une quelconque des revendications précédentes, comprenant en outre deux portions découpées à l'emporte-pièce (54) dans la région de pliage (50), de préférence positionnées à chaque point d'extrémité de la ligne de fragilité (52).

7. Emballage à deux unités (100) selon la revendication 6, dans lequel les deux portions découpées à l'emporte-pièce (54) sont concaves, en ayant de préférence un rayon de courbure R d'environ 0,2 mm à environ 6 mm, de préférence d'environ 0,4 mm à environ 5 mm, et mieux encore d'environ 0,5 mm à environ 4 mm.

8. Emballage à deux unités (100) selon l'une quelconque des revendications précédentes, dans lequel le film formable (40) est un plastique.

9. Emballage à deux unités (100) selon l'une quelconque des revendications précédentes, dans lequel les première et deuxième cavités (12, 22) retiennent un produit de consommation, et dans lequel les produits de consommation dans les première et deuxième cavités (12, 22) sont différents.

10. Emballage à deux unités (100) selon la revendication 9, dans lequel la première cavité (12) comprend une composition de soin personnel avec au moins un résorcinol et la deuxième cavité (22) comprend une composition de soin personnel comprenant au moins un précurseur ou dérivé d'acide rétinoïque,
dans lequel l'au moins un résorcinol est choisi parmi le 4-éthylrésorcinol, le 4-hexylrésorcinol, le 4-phényléthylrésorcinol, le 4-cyclopentylrésorcinol, le 4-cyclohexylrésorcinol, le 4-octylrésorcinol, et leurs mélanges,
dans lequel l'au moins un précurseur ou dérivé d'acide rétinoïque est choisi parmi le rétinol, le rétinal, le palmitate de rétinyle, le formiate de rétinyle, l'acétate de rétinyle, le propionate de rétinyle, le butyrate de rétinyle, le valérate de rétinyle, l'isovalérate de rétinyle, l'hexanoate de rétinyle, l'heptanoate de rétinyle, l'octanoate de rétinyle, le nonanoate de rétinyle, le décanoate de rétinyle, l'undécanoate de rétinyle, le taurate de rétinyle, le tridécanoate de rétinyle, le myristate de rétinyle, le pentadécanoate de rétinyle, l'heptadécanoate de rétinyle, le stéarate de rétinyle, l'isostéarate de rétinyle, le nonadécanoate de rétinyle, l'arachidonate de rétinyle, le béhénate de rétinyle, le linoléate de rétinyle, l'oléate de rétinyle, et leurs mélanges, et
sous réserve que l'au moins un résorcinol ne soit pas inclus dans la composition dans la deuxième cavité et que l'au moins un précurseur ou dérivé d'acide rétinoïque ne soit pas inclus dans la composition dans la première cavité.

11. Emballage à deux unités (100) selon la revendication 10, dans lequel la première cavité (12) et/ou la deuxième cavité (22) comprennent un composé hétéroaromatique fonctionnalisé, de préférence l'acide nicotinique, le niacinamide, un nicotinate, l'acide picolinique, le picolinamide, et leurs mélanges.

12. Emballage à deux unités (100) selon la revendication 9, dans lequel le produit de consommation est un produit de soin personnel.

13. Utilisation de l'emballage à deux unités (100) des revendications 1 à 8 pour conditionner deux produits de consommation différents, de préférence des produits de soin personnel, mieux encore des produits capillaires ou des compositions de soin personnel.

14. Méthode d'utilisation de l'emballage à deux unités (100) des revendications 1 à 8, comprenant :
la rupture de la ligne de fragilité (52) dans la région de pliage (50) ;
le pliage des premier et deuxième emballages coques (10, 20) ensemble de façon que les envers (15) sur chaque côté de la ligne de fragilité soient réunis, avec les première et deuxième cavités (12, 22) tournées vers l'extérieur ; et
le pressage des première et deuxième cavités (12, 22) des premier et deuxième emballages coques (10, 20) simultanément pour libérer un produit de consommation depuis les protubérances (30).
